# EUROPEAN PATENT SPECIFICATION

(11) **EP 3 870 188 B1**
(45) Date of publication and mention of the grant of the patent: **19.06.2024**
(21) Application number: 19806346.3
(22) Date of filing: 23.10.2019
(51) Int. Cl.: A61K 35/12, A61K 35/34, C12N 5/00, A61N 1/32, A61K 35/545, A61N 1/36, A61P 9/00, A61P 35/00, C12N 5/077

(54) **PREVENTION AND TREATMENT OF TERATOMA FORMATION IN STEM CELL-BASED THEREAPIES USING ALTERNATING ELECTRIC FIELDS**
VORBEUGUNG UND BEHANDLUNG DER TERATOMABILDUNG IN STAMMZELLENBASIERTEN THERAPIEN UNTER VERWENDUNG ALTERNIERENDEN ELEKTRISCHEN FELDERN
PRÉVENTION ET TRAITEMENT DE LA FORMATION DE TÉRATOMES DANS DES THÉAPIES À BASE DE CELLULES SOUCHES À L'AIDE DE CHAMPS ÉLECTRIQUES ALTERNATIFS

(30) Priority: 23.10.2018 US 201862749305 P
(43) Date of publication of application: 01.09.2021
(73) Proprietor: The Board of Trustees of the Leland Stanford Junior University, Stanford, CA 94305-2038 (US)
(72) Inventor: WARDAK, Mirwais, Stanford, California 94305 (US); CHANG, Edwin, Palo Alto, California 94304 (US); WU, Joseph C., Palo Alto, California 94304 (US); GAMBHIR, Sanjiv Sam, Portola Valley, California 94028 (US)
(74) Representative: Carpmaels & Ransford LLP
(86) International application number: PCT/US2019/057716
(87) International publication number: WO 2020/086753

(56) References cited:
- US-A1- 2017 009 210
- JEONG HO-CHANG ET AL: "Technical approaches to induce selective cell death of pluripotent stem cells", CMLS CELLULAR AND MOLECULAR LIFE SCIENCES, BIRKHAUSER VERLAG, HEIDELBERG, DE, vol. 74, no. 14, 28 February 2017 (2017-02-28), pages 2601-2611, XP036263813, ISSN: 1420-682X, DOI: 10.1007/S00018-017-2486-0 [retrieved on 2017-02-28]
- LISA A. FLANAGAN ET AL: "Unique Dielectric Properties Distinguish Stem Cells and Their Differentiated Progeny", STEM CELLS (MIAMISBURG), vol. 26, no. 3, 1 March 2008 (2008-03-01), pages 656-665, XP055662121, ISSN: 1066-5099, DOI: 10.1634/stemcells.2007-0810
- CHOO A B ET AL: "Selection against undifferentiated human embryonic stem cells by a cytotoxic antibody recognizing podocalyxin-like protein-1", STEM CELLS (MIAMISBURG),, vol. 26, no. 6, 1 June 2008 (2008-06-01), pages 1454-1463, XP002538481, ISSN: 1066-5099, DOI: 10.1634/STEMCELLS.2007-0576 [retrieved on 2008-03-20] cited in the application
- YU-TSEN LIN ET AL: "Elimination of undifferentiated human embryonic stem cells by cardiac glycosides", SCIENTIFIC REPORTS, vol. 7, no. 1, 13 July 2017 (2017-07-13), page 5289, XP055561081, DOI: 10.1038/s41598-017-05616-2 cited in the application
- HENG LIANG TAN ET AL: "mAb 84, a Cytotoxic Antibody that Kills Undifferentiated Human Embryonic Stem Cells via Oncosis", STEM CELLS (MIAMISBURG),, vol. 27, no. 8, 1 August 2009 (2009-08-01) , pages 1792-1801, XP008150204, ISSN: 1066-5099, DOI: 10.1002/STEM.109 [retrieved on 2009-04-30] cited in the application

## Description

### BACKGROUND

Regenerative medicine is a game-changing area of medicine which involves the process of creating living, functional tissues to repair or replace tissue or organ function lost due to age, disease, damage, or congenital defects. This field holds the promise of repairing or replacing damaged tissues and organs in the body by introducing outside cells, tissue, or even whole organs to integrate and become a part of tissues or replace whole organs. Importantly, regenerative medicine has the potential to solve the shortage of donor organs for patients who require life-saving organ transplantation.

One key to the success of regenerative medicine strategies has been the ability to isolate and generate stem cells, including pluripotent stem cells. Pluripotent stem cells, including embryonic stem cells (ESCs or ES cells) and induced pluripotent stem cells (iPSCs or iPS cells), are a leading candidate for cell-based therapies because of their capacity for unlimited self-renewal and their ability to differentiate into any cell type in the body, including whatever cell type is needed to replace tissue that is damaged by disease or injury. This type of treatment could be used, for example, to replace neurons damaged by stroke, spinal cord injury, Alzheimer's disease, Parkinson's disease, or other neurological disorders. Pancreatic cells grown to produce insulin could treat people with diabetes and heart cells could repair damage after a heart attack. This list could conceivably include any tissue that is injured or diseased. Stem cell treatments could also be used to help the body's own cells fight cancer by engineering, through stem cells, a cancer-fighting immune system.

Despite their therapeutic promise, an important safety concern for the clinical implementation of human pluripotent stem cells is their potential to form teratomas in vivo. Teratomas are complex tumors caused by the contamination of therapeutic stem cell-derived cells by residual pluripotent stem cells that escape the differentiation process. Even small amounts of undifferentiated pluripotent stem cells injected into tissues (e.g., 10,000 ES cells injected into the skeletal muscle) may result in teratomas. See, for example, Lee et al. (2009) Cell Cycle 8: 2608-2612. It is therefore essential to overcome this obstacle before stem cell therapies become acceptable for human use.

Several attempts have been made to selectively remove residual undifferentiated pluripotent stem cells from the pre-transplanted cells while sparing their differentiated progeny cells. These methods include the use of cytotoxic antibodies (Tan et al., 2009; Choo et al., 2008), specific antibody cell sorting (Tang et al., 2011; Fong et al., 2009), genetic manipulations including introduction of suicide genes (Blum et al., 2009; Schuldiner et al., 2003), pharmacological approaches (Lee et al., 2013; Ben-David et al. 2013; Lin et al., 2017), and radiation therapy (Lee et al., 2017). A description of different technical approaches to induce selective cell death of pluripotent stem cells is described in Jeong Ho-Chang et al., 2017. However, each of these methods has significant disadvantages, such as a high cost (cytotoxic antibodies and specific antibody cell sorting), variation among different lots (cytotoxic antibodies and specific antibody cell sorting), non-specific binding (cytotoxic antibodies), requirement of genetic manipulation and stable integration of toxic genes (genetic manipulation), time-consuming procedures (genetic manipulation, specific antibody cell sorting and cytotoxic antibodies), and use of ionizing radiation (radiation therapy). Although many studies have attempted to prevent teratoma formation from residual pluripotent stem cells, a clinically applicable strategy to eliminate teratoma formation has not yet been developed.

### SUMMARY OF THE INVENTION

One aspect of the invention is directed to a method of eliminating residual pluripotent stem cells from a batch of differentiated progeny cells in order to prevent the formation of teratomas in a stem cell-based therapy according to claim 1.

Embodiments of the invention are disclosed in the dependent claims.

### BRIEF DESCRIPTION OF THE DRAWINGS

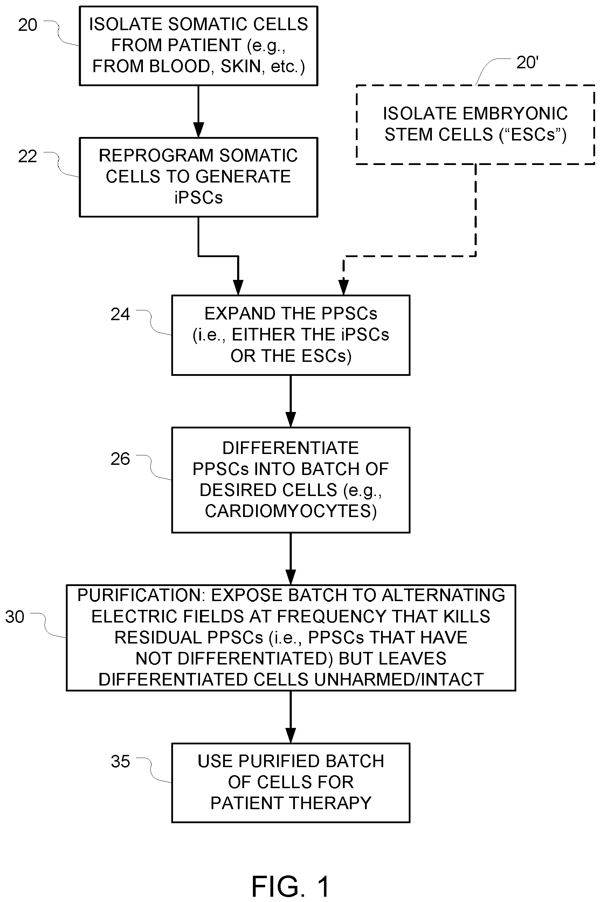

FIG. 1 is a flowchart depicting the steps of one exemplary approach for a stem cell-based therapy that dramatically reduces the risk of teratoma formation.

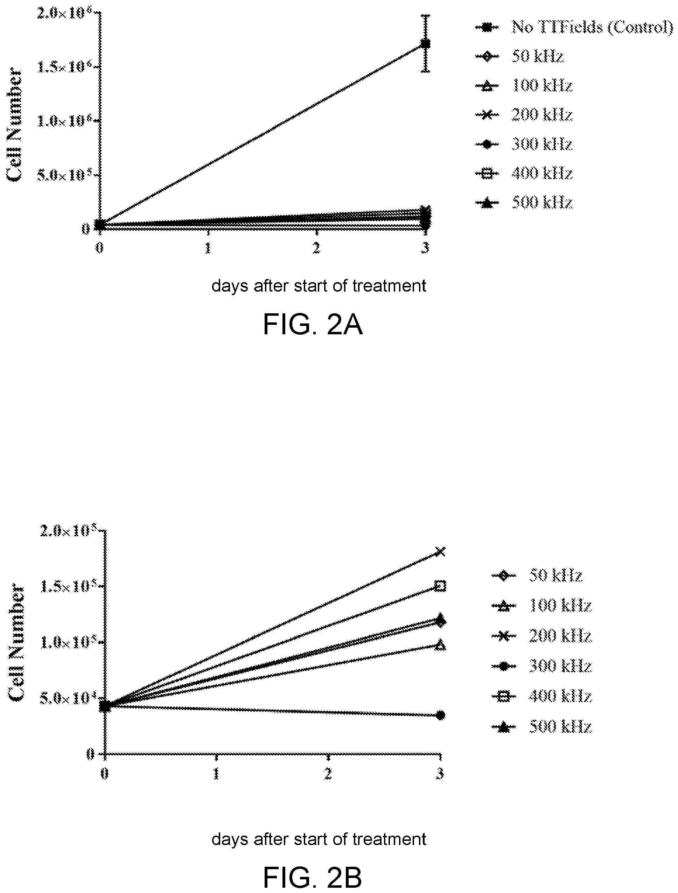

FIG. 2A depicts cell number counts as assessed by Trypan blue cell staining of the H7 human embryonic stem cells (H7-ESCs) that were exposed to alternating electric fields at different frequencies over time and compared to the non-exposed controls.
FIG. 2B is a zoomed-in version of the data shown in FIG. 2A of the H7-ESCs that were exposed to the alternating electric fields at the different frequencies over time.

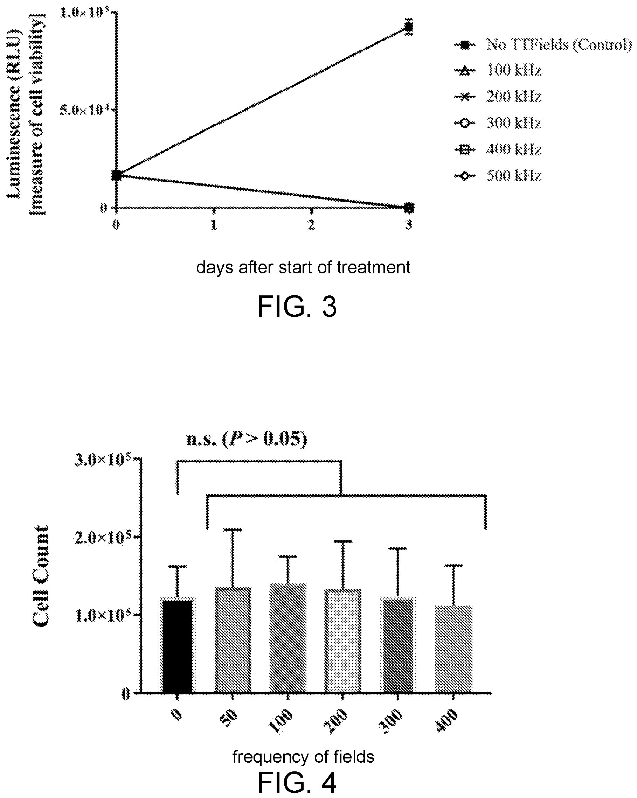

FIG. 3 depicts the cell viability of human ESCs (H7 line) over time when exposed to the alternating electric fields at the different frequencies and compared to the non-exposed controls. The luminescent output was positively correlated to the cell number (R²=0.942). RLU stands for relative luminescence unit.
FIG. 4 depicts cell counts as assessed by Trypan blue cell staining of ESC-derived cardiomyocytes after exposure to the alternating electric fields at the different frequencies and compared to the non-exposed control cardiomyocytes. There was no significant difference in the cardiomyocyte counts before and after the application of the alternating electric fields for any of the five frequencies that were tested.

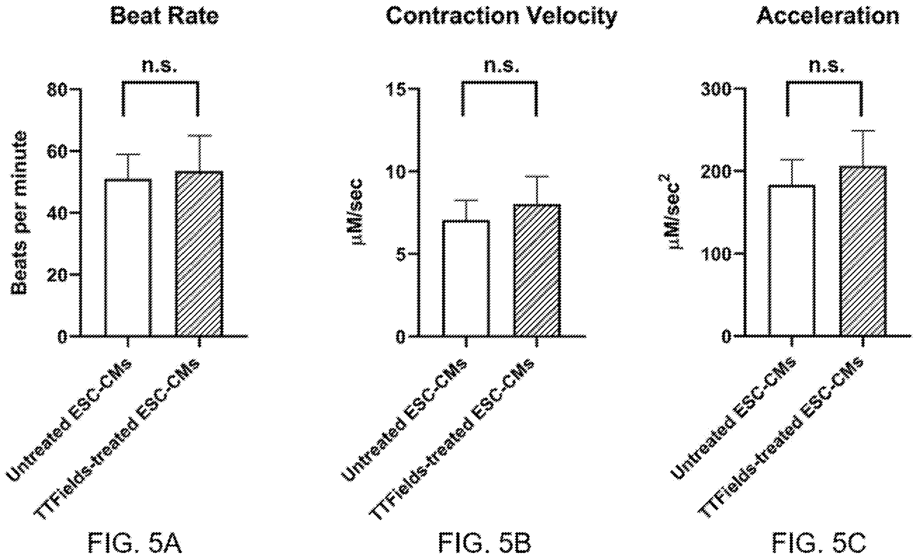

FIG. 5A depicts the results of a contractility assay that measured the beat rate of ESC-derived cardiomyocytes after exposure to the alternating electric fields and compared to the non-exposed (untreated) controls. The beat rate did not significantly differ between the ESC-CMs that were exposed to the alternating electric fields and those that were not exposed.
FIG. 5B depicts the results of a contractility assay that measured the contraction velocity of ESC-derived cardiomyocytes after exposure to the alternating electric fields and compared to the non-exposed (untreated) controls. The contraction velocity did not significantly differ between the ESC-CMs that were exposed to the alternating electric fields and those that were not exposed.
FIG. 5C depicts the results of a contractility assay that measured the acceleration of ESC-derived cardiomyocytes after exposure to the alternating electric fields and compared to the non-exposed (untreated) controls. The acceleration did not significantly differ between the ESC-CMs that were exposed to the alternating electric fields and those that were not exposed.

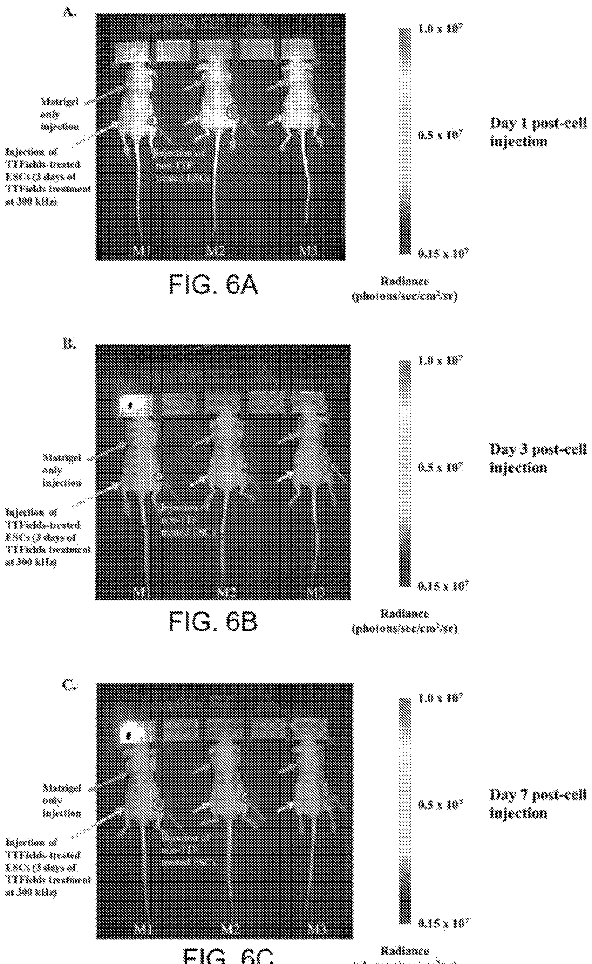

FIGS. 6A-6C depict. BLI signals at three different times post-cell injection of H7-ESCs in live mice. Note the drastic BLI signal difference in the alternating electric field-treated ESC injection side vs. the non-treated ESC injection side. There was absolutely no BLI signal detected in the left flanks of the mice that were injected with alternating electric field-treated ESCs.

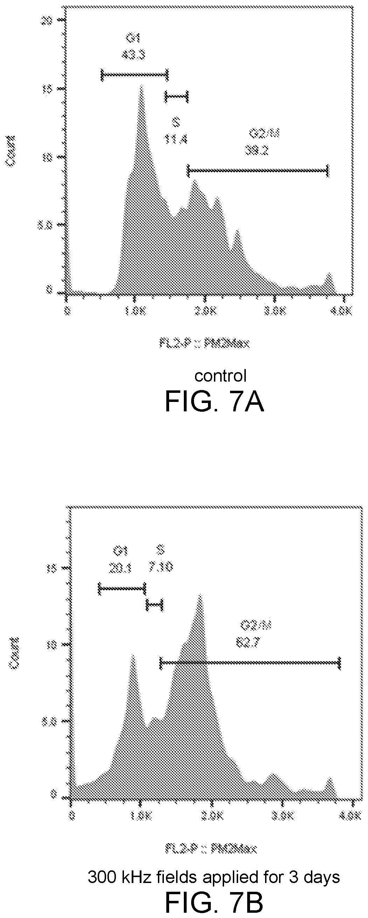

FIGS. 7A-7B depict the cell cycle analysis results for control ESCs that were not exposed to the alternating electric fields (FIG. 7A) and for ESCs that underwent 3 days of alternating electric field treatment at 300 kHz (FIG. 7B).

Various embodiments are described in detail below with reference to the accompanying drawings, wherein like reference numerals represent like elements.

### DESCRIPTION OF THE PREFERRED EMBODIMENTS

Eliminating the risk of residual pluripotent stem cells (which could give rise to teratomas) in prior stem cell-based therapies has previously been extremely difficult. The embodiments described herein use a novel approach to overcome this difficulty.

By "pluripotency" and pluripotent stem cells it is meant that such cells have the ability to differentiate into all types of cells in an organism. The term "induced pluripotent stem cell" encompasses pluripotent cells, that, like embryonic stem (ES) cells, can be cultured over a long period of time while maintaining the ability to differentiate into all types of cells in an organism, but that, unlike ES cells (which are derived from the inner cell mass of blastocysts), are derived from differentiated somatic cells, that is, cells that had a narrower, more defined potential and that in the absence of experimental manipulation could not give rise to all types of cells in the organism. iPS cells have an ESC-like morphology, growing as flat colonies with large nucleo-cytoplasmic ratios, defined borders and prominent nuclei. In addition, iPS cells express one or more key pluripotency markers known by one of ordinary skill in the art, including but not limited to alkaline phosphatase, SSEA3, SSEA4, Sox2, Oct3/4, Nanog, TRA160, TRA181, TDGF 1, Dnmt3b, FoxD3, GDF3, Cyp26a1, TERT, and Zfp42. In addition, the iPS cells are capable of forming teratomas. In addition, they are capable of forming or contributing to ectoderm, mesoderm, or endoderm tissues in a living organism.

FIG. 1 is a flowchart depicting the steps of one exemplary approach for a stem cell-based therapy that dramatically reduces the risk of teratoma formation. (Abbreviations: ESCs - embryonic stem cells, iPSCs - induced pluripotent stem cells, PPSCs - pluripotent stem cells.) The first four steps (i.e., steps 20-26) are similar to corresponding steps in prior art approaches for stem cell-based therapies. The improvement with respect to the prior art approaches is provided by the remaining steps (i.e., steps 30-35).

The entry point into the FIG. 1 flowchart depends on whether the process relies on induced pluripotent stem cells (iPSCs) or embryonic stem cells (ESCs). If the process relies on iPSCs, the process starts in step 20, where cells are isolated from the subject to obtain an initial cell population that will be reprogrammed to generate pluripotent stem cells from which therapeutic cells will be derived for stem cell-based therapy. Alternatively, cells may be extracted/isolated from a different donor. The cells may be initially isolated from the subject (or other donor), for example, by a skin sample or by drawing blood from that person.

A "starting cell population", or "initial cell population" refers to a somatic cell, usually a primary, or non-transformed, somatic cell, which undergoes nuclear reprogramming to pluripotency. The starting cell population may be of any mammalian species, but particularly including human cells. Sources of starting cell populations include individuals desirous of cellular therapy, individuals having a genetic defect of interest for study, and the like.

In some embodiments, human cells obtained from a subject for regenerative purposes may be chosen from any human cell type, including fibroblast cells, adipose tissue cells, mesenchymal cells, bone marrow cells, stomach cells, liver cells, epithelial cells, nasal epithelial cells, mucosal epithelial cells, follicular cells, connective tissue cells, muscle cells, bone cells, cartilage cells, gastrointestinal cells, splenic cells, kidney cells, lung cells, testicular cells, nervous tissue cells, etc. In some embodiments, the human cell type is a fibroblast, which may be conveniently obtained from a subject by a punch biopsy.

Next, in step 22, the initial cell population is reprogrammed to generate induced pluripotent stem cells (e.g., iPSCs). This may be implemented using any of a variety of approaches that will be apparent to persons skilled in the relevant arts. As used herein, "reprogramming factors" refers to one or more, i.e. a cocktail, of biologically active factors that act on a cell to alter transcription, thereby reprogramming a cell to multipotency or to pluripotency. Reprogramming factors may be provided to the cells, e.g. cells from an individual with a family history or genetic make-up of interest for heart disease such as fibroblasts, adipocytes, etc.; individually or as a single composition, that is, as a premixed composition, of reprogramming factors. The factors may be provided at the same molar ratio or at different molar ratios. The factors may be provided once or multiple times in the course of culturing the cells. In some embodiments the reprogramming factor is a transcription factor, including without limitation, Oct3/4; Sox2; Klf4; c-Myc; Nanog; and Lin-28.

Somatic cells are contacted with reprogramming factors, as defined above, in a combination and quantity sufficient to reprogram the cell to pluripotency. Reprogramming factors may be provided to the somatic cells individually or as a single composition, that is, as a premixed composition, of reprogramming factors. In some embodiments the reprogramming factors are provided as a plurality of coding sequences on a vector.

Optionally, genes may be introduced into the somatic cells or the iPS cells derived therefrom for a variety of purposes, e.g. to replace genes having a loss of function mutation, provide marker genes, etc. Alternatively, vectors are introduced that express antisense mRNA or ribozymes, thereby blocking expression of an undesired gene. Other methods of gene therapy are the introduction of drug resistance genes to enable normal progenitor cells to have an advantage and be subject to selective pressure, for example the multiple drug resistance gene (MDR), or anti-apoptosis genes, such as bcl-2. Various techniques known in the art may be used to introduce nucleic acids into the target cells, e.g. electroporation, calcium precipitated DNA, fusion, transfection, lipofection, infection and the like, as discussed above. The particular manner in which the DNA is introduced is not criti cal.

Note that instead of beginning with a somatic cell that has been isolated from the subject or other donor and reprogrammed to form an induced pluripotent stem cell (as described above in connection with steps 20-22), the pluripotent stem cell(s) that are used in the subsequent steps may be obtained using an alternative approach. Examples include obtaining embryonic stem cells using any conventional approach for obtaining such cells. In this situation, the entry point into the FIG. 1 flowchart would be step 20', where the ESCs are isolated.

Step 24 is performed after the pluripotent stem cells (PPSCs) have been obtained as described above (i.e., using steps 20-22 in the case of induced pluripotent stem cells, or step 20' in the case of embryonic stem cells). In this step 24, the initial quantity of pluripotent stem cells is expanded using any of a variety of approaches that will be appreciated by persons skilled in the relevant arts. For example, the initial quantity of pluripotent stem cells may be cultured and fed an appropriate nutrient medium until a batch of pluripotent stem cells that is large enough for the intended application has been obtained.

Next, in step 26, the batch of pluripotent stem cells is differentiated to form a batch of specialized cells with the desired type of differentiation, which will depend on the nature of the treatment that will ultimately be needed. For example, if the end goal is to introduce cardiomyocytes into a patient with heart disease, the differentiation process in step 26 should differentiate the pluripotent stem cells into cardiomyocytes.

Examples of differentiated cells include any differentiated cells from ectodermal (e.g., neurons and fibroblasts), mesodermal (e.g., cardiomyocytes), or endodermal (e.g., pancreatic cells) lineages. The differentiated cells may be one or more: cholinergic, serotonergic, GABAergic, glutamatergic neuronal cells, pancreatic beta cells, neural stem cells, neurons (e.g., dopaminergic neurons), retinal ganglion cells, photoreceptor cells (rods and cones), retinal pigment epithelial cells, oligodendrocytes, oligodendrocyte progenitor cells, hepatocytes, hepatic stem cells, chondrocytes, bone cells, connective tissue cells, glial cells, astrocytes, myocytes, hematopoietic cells, egg or sperm cells, pacemaker cells or cardiomyocytes.

Although the methods described herein are not limited to producing cardiomyocytes, the following example of differentiating a batch of pluripotent stem cells into a batch of cardiomyocytes is provided for illustrative purposes. Pluripotent cells can be differentiated into somatic cells, including without limitation cardiac muscle cells. Inhibition of bone morphogenetic protein (BMP) signaling may result in the generation of cardiac muscle cells (or cardiomyocytes), see, e.g., Yuasa et al., (2005), Nat. Biotechnol., 23(5):607-11. Thus, in one embodiment pluripotent cells are cultured in the presence of noggin for from about two to about six days, e.g., about 2, about 3, about 4, about 5, or about 6 days, prior to allowing formation of an embryoid body, and culturing the embryoid body for from about 1 week to about 4 weeks, e.g., about 1, about 2, about 3, or about 4 weeks.

Cardiomyocyte differentiation may be promoted by including cardiotropic agents in the culture, such as activin A and/or bone morphogenetic protein-4 (see the examples herein, Xu et al. Regen Med. 2011 Jan;6(1):53-66; Mignone et al. Circ J. 2010 74(12):2517-26; Takei et al. Am J Physiol Heart Circ Physiol. 2009 296(6):H1793-803). Examples of such protocols also include, for example, addition of a Wnt agonist, such as Wnt 3A, optionally in the presence of cytokines such as BMP4, VEGF and Activin A; followed by culture in the presence of a Wnt antagonist, such a soluble frizzled protein. However, any suitable method of inducing cardiomyocyte differentiation may be used, for example, Cyclosporin A described by Fujiwara et al. PLoS One. 2011 6(2):e16734; Dambrot et al. Biochem J. 2011 434(1):25-35; equiaxial cyclic stretch, angiotensin II, and phenylephrine (PE) described by Foldes et al. J Mol Cell Cardiol. 2011 50(2):367-76; ascorbic acid, dimethylsulfoxide and 5-aza-2'-deoxycytidine described by Wang et al. Sci China Life Sci. 2010 53(5):581-9, endothelial cells described by Chen et al. J Cell Biochem. 2010 111(1):29-39, and the like.

The cells are harvested at an appropriate stage of development, which may be determined based on the expression of markers and phenotypic characteristics of the desired cell type (e.g. at from about 1 to 4 weeks). Cultures may be empirically tested by staining for the presence of the markers of interest, by morphological determination, etc. The cells are optionally enriched before or after the positive selection step by drug selection, panning, density gradient centrifugation, etc.

A "cardiomyocyte precursor" is defined as a cell that is capable of giving rise to progeny that include cardiomyocytes. Phenotypes of cardiomyocytes that arise during development of the mammalian heart can be distinguished: primary cardiomyocytes; nodal cardiomyocytes; conducting cardiomyocytes and working cardiomyocytes. All cardiomyocytes have sarcomeres and a sarcoplasmic reticulum (SR), are coupled by gap junctions, and display automaticity. Cells of the primary heart tube are characterized by high automaticity, low conduction velocity, low contractility, and low SR activity. This phenotype largely persists in nodal cells. In contrast, atrial and ventricular working myocardial cells display virtually no automaticity, are well coupled intercellularly, have well developed sarcomeres, and have a high SR activity. Conducting cells from the atrioventricular bundle, bundle branches and peripheral ventricular conduction system have poorly developed sarcomeres, low SR activity, but are well coupled and display high automaticity.

For α-Mhc, β-Mhc and cardiac Troponin I and slow skeletal Troponin I, developmental transitions have been observed in differentiated ES cell cultures. Expression of Mlc2v and Anf is often used to demarcate ventricular-like and atrial-like cells in ES cell cultures, respectively, although in ES-derived cells (ESDCs), Anf expression does not exclusively identify atrial cardiomyocytes and may be a general marker of the working myocardial cells.

At this point in the process (i.e., the end of step 26), we have a batch of differentiated progeny cells of the desired type (e.g., cardiomyocytes). But because the differentiated progeny cells were created using a process that involves pluripotent stem cells, it is highly probable that not all of the pluripotent stem cells have been differentiated into the desired type of cell. This means that there is a high probability that residual pluripotent stem cells remain mixed in with the batch of differentiated cells. Therefore, before the batch of differentiated progeny cells is used for therapeutic purposes, the batch of differentiated progeny cells should be purified by eliminating residual pluripotent stem cells that remain mixed in with the batch of differentiated cells (preferably to the maximum extent possible). This will prevent the formation of teratoma tumors when the batch of differentiated progeny cells is eventually used in a stem cell-based therapy.

Step 30 is where this purification occurs. More specifically, in step 30, the batch of differentiated progeny cells along with any residual pluripotent stem cells mixed therein is exposed to an alternating electric field for a period of time. The alternating electric field has frequency and field strength characteristics. The frequency and field strength of the alternating electric field are such that as a result of being exposed to the alternating electric field for the period of time, the pluripotent stem cells die off, resulting in a purified batch of differentiated progeny cells that is rendered safe for subsequent use in the stem cell-based therapy. Meanwhile, the frequency and field strength of the alternating electric field are also such that the differentiated cells that are exposed to the alternating electric field for the period of time remain substantially unharmed.

Importantly, it is not necessary to kill all of the pluripotent stem cells to render the purified batch safe. Instead, a small number of pluripotent stem cells can remain, as long as that number is small enough such that using the purified batch of differentiated progeny cells in a stem cell-based therapy will not result in an undue risk of teratoma formation. An example of this in numeric terms in the context of mice would be fewer than 10,000 pluripotent stem cells injected into skeletal muscle and fewer than 100,000 pluripotent stem cells injected into the myocardium. In some preferred embodiments, the number of pluripotent stem cells is reduced to the maximum extent possible (e.g., to fewer than 1000 or fewer than 100 pluripotent stem cells).

A set of experiments was performed to determine suitable exemplary frequencies and field strengths for the electric field. Those experiments revealed that frequencies between 50 kHz and 500 kHz and field strengths of 1-4 V/cm were effective for purifying the differentiated progeny cells by eliminating the rapidly dividing pluripotent stem cells from the starting pre-purified batch.

Tumor-treating fields (TTFields) is an FDA-approved therapy for solid tumors that operates by delivering low-intensity (e.g., 1-4 V/cm) alternating electric fields within the intermediate-frequency range (e.g., 50-500 kHz) to the tumors. And any of the known approaches for applying TTFields to a culture in vitro or to a subject in vivo may be used to apply alternating electric fields to the batch of differentiated progeny cells mixed among residual pluripotent stem cells in order to purify the batch of differentiated cells and thereby prevent formation of teratoma tumors. Because TTFields are alternating electric fields, one example of an approach that may be used for eliminating pluripotent stem cells from a batch of differentiated progeny cells that contains residual pluripotent stem cells is to use the Novocure INOVITRO^{™} system. The INOVITRO^{™} system is comprised of a TTFields generator controlled by software, and base plates with ultra-high dielectric constant ceramic petri dishes. The INOVITRO^{™} system allows researchers to set target TTFields intensity and frequency in each ceramic dish, and was used for performing the in the vitro experiments described herein.

The TTFields in vitro application system has a core unit of a high dielectric-constant ceramic (i.e., lead magnesium niobate-lead titanate [PMN-PT]) Petri dish. Two pairs of electrodes are printed perpendicularly in the circuit board glued to the bottom of a TTFields ceramic dish to allow for the application of electric fields from two perpendicular directions parallel to the plane of the dish. Such a configuration optimizes the proportion of cells in metaphase-spread that will be exposed to the alternating electric fields. As cell division may occur at any time, prolonged exposure to the electrical fields is required for maximal effect.

Electric currents through the electrodes are achieved by fitting the ceramic dishes onto a base plate that is in turn hooked to a power box connected to a sinusoidal waveform generator and an amplifier. This set-up allows for TTFields application in the frequency range of 50-500 kHz with electric field strengths of 1-4 V/cm. In order to dissipate excessive heat, the TTFields dishes are kept inside a temperature regulated incubator pre-set to 20-27° C. Temperature of the media within the well of a ceramic dish was controlled by constantly monitoring the dish temperature through temperature probes printed onto the circuit board at the bottom of the dish. Temperature and electric field strengths were kept constant through computer-controlled adjustments in the voltage and current that were applied to the bottom of the circuit board by the INOVITRO^{™} system.

A typical workflow for experiments for cardiac stem cell therapy using the INOVITRO^{™} system will now be described. Depending on the nature of the experiment, either (a) ESC-derived cardiomyocytes or (b) ESCs were seeded onto Matrigel coated coverslips. The coverslips were next transferred to ceramic dishes which were filled with appropriate media (~2 mL) in advance. Cover lids were placed on the ceramic dishes to mitigate evaporation during the TTFields experiment. Ceramic dishes were then installed onto the TTFields base plate. The experimental dishes placed on the base plates were transferred to a special incubator for the alternating electric field conditions. The base plates were then connected with a flat cable connector to the TTFields generator. After the treatment settings were set using the Inovitro^{™} software, the experiment was started. TTFields set anywhere from 1-4 V/cm were applied through the Inovitro^{™} power generator. The TTFields frequencies selected included 50 kHz, 100 kHz, 200 kHz, 300 kHz, 400 kHz and 500 kHz. Incubation temperatures spanned 20-27° C with a target temperature of 37° C for the ceramic dishes upon application of the TTFields. The culture medium was exchanged manually every 24 h throughout the experiments. Treatment duration lasted anywhere from 3-4 days, after which coverslips were removed and cell count or cell viability assays were performed. Control dishes were placed in a separate tissue culture incubator set to 37° C, 95% air and 5% CO₂. Unless otherwise mentioned, all experiments were done in triplicate samples per condition.

For each experiment, the same number of cells were seeded onto each coverslip. Depending on the individual experiment, a set seeding density of 50,000 - 500,000 cells was used per coverslip.

Importantly, while the INOVITRO^{™} system was used to perform the in vitro experiments described herein, the methods described herein are not limited to applying the alternating electric field using the INOVITRO^{™} system. To the contrary, the alternating electric fields may be applied using any of a variety of alternative approaches, including but not limited to systems that are similar to the INOVITRO^{™} system, but scaled-up in size to increase the quantity of cells to which the alternating electric fields are applied.

The genus of pluripotent stem cells includes both embryonic stem cells (ESCs) and induced pluripotent stem cells (iPSCs). And although the experiments described herein were performed on embryonic stem cells, the results should be applicable to other types of pluripotent stem cells as well.

In some of the experiments, H7-ESCs were transfected with a lentivirus vector that expresses Luciferase and Tomato Red under the EF1a promoter. Transfecting the embryonic stem cells with this lentivirus vector was advantageous because the tomato red causes the ESCs to become visible when illuminated by light of the appropriate wavelength, and the luciferase causes the ESCs to become visible when luciferin is available to the cell.

FIGS. 2A and 2B depict the results of a first experiment that measured the cell number counts of H7 human embryonic stem cells over time when exposed to alternating electric fields at 50 kHz, 100 kHz, 200 kHz, 300 kHz, 400 kHz, and 500 kHz, as well as a control in which alternating electric fields were not applied. (FIGS. 2A and 2B depict the same data at different Y-axis scales.) The non-treated control ESCs had a >3600% increase in their cell number after 3 days of being in the incubator (i.e., there was a jump from -45,000 ESCs at Day 0 [baseline] to ~1.7 million cells at Day 3). (FIG. 2B) The frequency which showed the highest ESC killing among the frequencies tested was 300 kHz. It is believed that prolonged exposure to the alternating electric fields would eradicate virtually all of the ESCs.

FIG. 3 depicts the cell viability of ESCs over time when exposed to alternating electric field at different frequencies. Viability was determined based on the luminescent signal obtained from the Cell Titer Glo assay at baseline before the start of alternating electric field therapy and at 3 days after the start of alternating electric field therapy. The non-treated control ESCs had a >450% increase in their luminescent output after 3 days of being in the incubator. However, there was virtually a complete eradication of ESCs which had alternating electric fields applied to them for 3 days at all five of the shown frequencies. (Note that the data points in FIG. 3 for 100 kHz, 200 kHz, 300 kHz, 400 kHz, and 500 kHz are all overlapping at a value that is very close to zero.) The frequency which showed the highest ESC killing was 300 kHz (>99.9% cell killing when compared to its baseline starting value). Cell number correlated with luminescent output (R² = 0.942). RLU stands for relative luminescence unit.

In another experiment, brightfield images were also used to observe the effects of alternating electric fields on embryonic stem cells. In this experiment, brightfield images of dishes of ESCs were obtained at baseline before the start of the experiment. Some dishes were exposed to alternating electric fields at a frequency of 300 kHz for three days, while other dishes (i.e., the control dishes) were not exposed to alternating electric fields for the same period of time. At the end of the experiment, visual observation of the brightfield images of the dishes revealed that the quantity of embryonic stem cells rapidly increased in the dishes that were not exposed to alternating electric fields, but appeared to be completely eradicated in the dishes that were exposed to alternating electric fields.

These experiments show that exposing pluripotent stem cells to alternating electric fields at the frequencies noted above causes the pluripotent stem cells to die.

Additional experiments were performed to determine what happens when differentiated cells that were derived from pluripotent stem cells are exposed to alternating electric fields at the same frequencies. More specifically, in these experiments dishes of ESC-derived cardiomyocytes (ESC-CMs) were visually observed before and after alternating electric fields were applied to those cells for three days at five different frequencies (50, 100, 200, 300, and 400 kHz). In addition, control dishes were visually observed before and after a three day time period during which alternating electric fields were not applied. Visual observation of the dishes did not reveal a noticeable significant difference in the cardiomyocyte wells before and after the application of the alternating electric fields.

In addition to visual observation, cell counting was also performed, and FIG. 4 depicts the results of cell counting of ESC-derived cardiomyocytes (ESC-CMs) after exposure to alternating electric field at different frequencies. The cell counting was done at the end of the three day period using a Trypan blue cell-counting assay. The cell counting revealed that the difference in the cardiomyocyte counts before and after the application of the alternating electric fields was not statistically significant for any of the five frequencies that were tested.

The ESC-derived cardiomyocytes were also seen beating both before and after the alternating electric fields were applied, indicating an intact functional phenotype (i.e., there were no harmful effects caused by the alternating electric field).

A decrease in the beat-to-beat variation in the ESC-derived cardiomyocytes after exposure to the alternating electric fields was also observed, showing that the improved electromechanical properties observed might be indicative of a more mature cardiomyocyte profile.

FIGS. 5A-5C depict the results of a contractility assay that measured the beat rate, contraction velocity, and acceleration of ESC-derived cardiomyocytes after exposure to alternating electric fields at 300 kHz, as well as control ESC-CMs that were not exposed to alternating electric fields. No statistically significant difference in the beat rate, contraction velocity, and acceleration of the cardiomyocytes were found between the ESC-CMs that were exposed to the alternating electric fields and those that were not exposed. This provides additional evidence that the cardiomyocytes were not harmed by the alternating electric fields.

Collectively, these experiments show that exposing a batch of stem cell-derived differentiated cells (e.g., cardiomyocytes) to alternating electric fields at the frequencies noted above leaves those cells substantially unharmed. Notably, the results observed for the differentiated progeny cells (which were not harmed by the alternating electric field, see e.g., FIG. 4) stand in sharp contrast with the results observed for the stem cells (which died when they were exposed to the alternating electric field, see e.g., FIG. 3).

FIGS. 6A-6C depict BLI signals at three different time points post-cell injection (FIG. 6A: Day 1, FIG. 6B: Day 3 and FIG. 6C: Day 7) of -500,000 human H7-ESCs in the right flanks of the mice. BLI signal at the three different time points is also shown for the alternating electric field-treated ESCs (3 days of alternating electric field treatment at 300 kHz) that were injected in the left flanks of the mice, and the matrigel only injections in the left shoulder blades of the mice (which served as our control). Note the drastic BLI signal difference in the alternating electric field-treated ESC injection side vs. the non-treated ESC injection side. There was absolutely no BLI signal detected in the left flanks of the mice that were injected with alternating electric field-treated ESCs. The luminescence signal was quantified in radiance units of photons per second per square centimeter per steradian (photons/sec/cm²/sr). This shows that exposing pluripotent stem cells to alternating electric fields reduces the number of pluripotent stem cells to the point where they do not form teratomas in vivo.

FIGS. 7A-7B depict cell cycle analysis results showing that in comparison to non-treated control ESCs (FIG. 7A), the ESCs that underwent 3 days of alternating electric field treatment at 300 kHz (FIG. 7B) were arrested in the G2/M checkpoint of the cell cycle. This gives important insights into mechanisms of action of the alternating electric field against rapidly dividing cells such as stem cells and tumor cells.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, cats, cows, etc. In some preferred embodiments, the mammal is human.

This application describes methods of eliminating residual pluripotent stem cells from a batch of differentiated progeny cells in order to prevent the formation of teratoma tumors in a stem cell-based therapy. In some embodiments, the population of cells purified by the methods described herein are purified to the point where less than 1 in 10⁷ cells have the properties of a pluripotent cell. In some embodiments, the population of cells purified by the methods described herein are purified to the point where there are fewer than 100,000 pluripotent stem cells. In some embodiments, the population of cells purified by the methods described herein are purified to the point where there are fewer than 10,000 pluripotent stem cells. In some embodiments, the population of cells purified by the methods described herein are purified to the point where there are fewer than 1,000 pluripotent stem cells. In some embodiments, the population of cells purified by the methods described herein are purified to the point where there are fewer than 100 pluripotent stem cells.

For in vitro embodiments, the population of cells may include any population suspecting of comprising a mixture of differentiated and pluripotent cells. Of particular interest are cultures of differentiated cells prepared as therapeutic agents, e.g. cells differentiated into a pathway of interest, where the differentiated cells were derived from stem cells, and where residual stem cells may be present in the batch of differentiated cells.

For elimination of pluripotent cells from a batch of differentiated progeny cells, an appropriate solution may be used for dispersion or suspension. Such solution will generally be a balanced salt solution, e.g. normal saline, PBS, Hank's balanced salt solution, etc., conveniently supplemented with fetal calf serum or other naturally occurring factors, in conjunction with an acceptable buffer at low concentration, generally from 5-25 mM. Convenient buffers include HEPES, phosphate buffers, lactate buffers, etc..

The pluripotent cells may be eliminated from the complex mixture of cells by suspending the cells in medium, and optionally allowing the cells to adhere to a surface. The cells are then subjected to low-intensity alternating electric fields within the intermediate-frequency range (50-500 kHz) for a period of time.

In some embodiments the frequency of the alternating electric field is from about 200 kHz to about 400 kHz, from about 250 kHz to about 350 kHz, and may be around 300 kHz. In some embodiments the field is at least 1 V/cm. In some embodiments, the field is between 1 and 4 V/m. In other embodiments combinations of field strengths are applied, for example combining two or more frequencies at the same time, and/or applying two or more frequencies at different times.

The exposure may last for at least 12 hours, at least 24 hours, at least 36 hours, at least 48 hours, or at least 72 hours or more.

In some embodiments the cell culture comprises an artificial organ cultured in vitro, for example where one or more different tissue types or tissue layers are co-cultured, where the artificial organ is treated by exposure to alternating electric fields for the elimination of the pluripotent cells before the organ is implanted into the body.

In some embodiments the alternating electric fields are combined with an effective dose of an agent that targets dividing cells, which include without limitation alkylating agents, and the like. Alkylating agents are known to act through the alkylation of macromolecules such as the DNA of cancer cells, and are usually strong electrophiles. This activity can disrupt DNA synthesis and cell division. Examples of alkylating reagents suitable for use herein include nitrogen mustards and their analogues and derivatives including, cyclophosphamide, ifosfamide, chlorambucil, estramustine, mechlorethamine hydrochloride, melphalan, and uracil mustard. Other examples of alkylating agents include alkyl sulfonates (e.g. busulfan), nitrosoureas (e.g. carmustine, lomustine, and streptozocin), triazenes (e.g. dacarbazine and temozolomide), ethylenimines/methylmelamines (e.g. altretamine and thiotepa), and methylhydrazine derivatives (e.g. procarbazine). Included in the alkylating agent group are the alkylating-like platinum-containing drugs comprising carboplatin, cisplatin, and oxaliplatin.

Additional agents can include, but are not limited to, analogues and derivatives of folic acid, pyrimidines, purines, and cytidine. Members of the folic acid group of agents suitable for use herein include, but are not limited to, methotrexate (amethopterin), pemetrexed and their analogues and derivatives. Pyrimidine agents suitable for use herein include, but are not limited to, cytarabine, floxuridine, fluorouracil (5-fluorouracil), capecitabine, gemcitabine, and their analogues and derivatives. Purine agents suitable for use herein include, but are not limited to, mercaptopurine (6-mercaptopurine), pentostatin, thioguanine, cladribine, and their analogues and derivatives. Cytidine agents suitable for use herein include, but are not limited to, cytarabine (cytosine arabinodside), azacitidine (5-azacytidine) and their analogues and derivatives.

Antimitotic agents suitable for use herein include, but are not limited to, vinca alkaloids like vinblastine, vincristine, vindesine, vinorelbine, and their analogues and derivatives, and podophyllotoxins, which include, but are not limited to etoposide, teniposide, and their analogues and derivatives. Antineoplastic agents suitable for use herein include, but are not limited to, belomycin, dactinomycin, doxorubicin, idarubicin, epirubicin, mitomycin, mitoxantrone, pentostatin, plicamycin, and their analogues and derivatives. Camptothecin analogues and derivatives which are suitable for use herein and include camptothecin, topotecan, and irinotecan.

The number of pluripotent cells remaining in a population may be assessed, e.g. culturing for the presence of teratomas forming cells in an aliquot of the population, or by analysis for markers of pluripotency, e.g. by staining for the presence of markers for example, Oct-4, Sox2, Nanog, KLF4, TRA-1-60/TRA-1-81/TRA-2-54, SSEA1, SSEA4, etc. For example, an aliquot of cells may be stained with antibodies that bind to one or more of such markers indicative of pluripotency. The antibodies are added to a suspension of cells, and incubated for a period of time sufficient to bind the available cell surface antigens, and the cells that bind to such antibodies are quantitated.

As defined above, the differentiated progeny cells that are exposed to the alternating electric field remain substantially unharmed. But note that this is to be analyzed from an overall perspective, and a small portion of the differentiated progeny cells may die. Preferably, less than 5% of the differentiated progeny cells will die. In alternative embodiments, less than 10%, 15%, or 25% of the differentiated progeny cells will die. In other words, there may be some killing of differentiated progeny cells, but it is desirable to have at least 75% viability of the differentiated progeny cells, at least 85%, at least 90%, at least 95% or more following the purification process.

The purified batch of differentiated progeny cells, which lack the residual pluripotent stem cells, may be resuspended in any appropriate medium that maintains the viability of the cells. Various media are commercially available and may be used according to the nature of the cells, including dMEM, HBSS, dPBS, RPMI, Iscove's medium, etc., frequently supplemented with fetal calf serum.

The purified batch of differentiated progeny cells may be used immediately. Alternatively, the cells may be frozen at liquid nitrogen temperatures and stored for long periods of time, being thawed and capable of being reused. In such cases, the cells will usually be frozen in 10% DMSO, 50% serum, 40% buffered medium, or some other such solution as is commonly used in the art to preserve cells at such freezing temperatures, and thawed in a manner as commonly known in the art for thawing frozen cells.

Returning to FIG. 1, the purified batch of differentiated progeny cells may be used therapeutically for treating a subject in step 35 or used for diagnostic purposes. The therapy may be directed at treating the cause of the disease; or alternatively, the therapy may be to treat the effects of the disease or condition. The induced cells may be transferred to, or close to, an injured site in a subject; or the cells can be introduced to the subject in a manner allowing the cells to migrate, or home, to the injured site. The transferred cells may advantageously replace the damaged or injured cells and allow improvement in the overall condition of the subject. In some instances, the transferred cells may stimulate tissue regeneration or repair.

The differentiated progeny cells may be transferred to subjects suffering from a wide range of diseases or disorders. Degenerative heart diseases such as ischemic cardiomyopathy, conduction disease, and congenital defects could benefit from regenerative cell therapies, see, e.g. Janssens et al., (2006), Lancet, 367:113-121.

In some embodiments the purified batch of differentiated progeny cells comprises cardiomyocytes differentiated in vitro from a pluripotent stem cell population. It was found that there is a decrease in the beat-to-beat variation in ESC-derived cardiomyocytes after treatment with alternating electric fields, demonstrating improved electromechanical properties indicative of a more mature cardiomyocyte profile. In some embodiments cardiomyocytes exhibit enhanced structural and functional maturation, relative to similarly differentiated cardiomyocytes not exposed to alternating electric fields.

In those embodiments that involve cardiomyocytes, a purified batch of differentiated cardiomyocyte or cardiomyocyte progenitor cell population can be administered to the heart of the subject in need thereof by injection. In one embodiment, the injection is intramyocardial. One of skill in the art would be well aware of advantages of delivering cardiac stem cells by intramyocardial injection as the heart is a functioning muscle. Intramyocardial injection minimizes the loss of the injected cells due to the contracting movements of the heart. Alternatively, the treated cells can be administered by injection transendocardially or trans-epicardially. In one embodiment, a catheter-based approach is used to deliver the trans-endocardial injection. The use of a catheter precludes more invasive methods of delivery wherein the opening of the chest cavity would be necessitated. As one skilled in the art would appreciate, optimum time of recovery would be allowed by the more minimally invasive procedure. A catheter approach can involve the use of such techniques as the NOGA catheter or similar systems. The NOGA catheter system facilitates guided administration by providing electromechanic mapping of the area of interest, as well as a retractable needle that can be used to deliver targeted injections or to bathe a targeted area with a therapeutic. The methods described herein can be performed through the use of such a system to deliver injections. One of skill in the art will recognize alternate systems that also provide the ability to provide targeted treatment through the integration of imaging and a catheter delivery system that can be used with the methods described herein. Information regarding the use of NOGA and similar systems can be found in, for example, Sherman (2003) Basic Appl. Myol. 13: 11-14; Patel et al (2005) The Journal of Thoracic and Cardiovascular Surgery 130:1631-38; and Perrin et al. (2003) Circulation 107: 2294-2302. In another embodiment, the stem cell-derived cardiac cells can be administered by an intracoronary route of administration. One of skill in the art will recognize other useful methods of delivery or implantation which can be utilized with the methods described herein, including those described in Dawn et al. (2005) Proc. Natl. Acad. Sci. USA 102, 3766-3771.

Subjects suffering from neurological diseases or disorders benefit from regenerative cell therapies. In some approaches, the purified batch of differentiated progeny cells are neural stem cells or neural cells transplanted to an injured site to treat a neurological condition, e.g., Alzheimer's disease, Parkinson's disease, multiple sclerosis, cerebral infarction, spinal cord injury, or other central nervous system disorder, see, e.g., Morizane et al., (2008), Cell Tissue Res., 331(1):323-326; Coutts and Keirstead (2008), Exp. Neurol., 209(2):368-377; Goswami and Rao (2007), Drugs, 10(10):713-719.

Endothelial cells are useful in improving vascular structure and function, enhancing angiogenesis, and improving perfusion, e.g. in peripheral arterial disease. Pancreatic islet cells (or primary cells of the islets of Langerhans) may be transplanted into a subject suffering from diabetes (e.g., diabetes mellitus, type 1), see e.g., Burns et al., (2006) Curr. Stem Cell Res. Ther., 2:255-266. In some embodiments, pancreatic beta cells are transplanted into a subject suffering from diabetes (e.g., diabetes mellitus, type 1). In other examples, hepatic cells or progenitors are transplanted into a subject suffering from a liver disease, e.g., hepatitis, cirrhosis, or liver failure.

Hematopoietic cells or hematopoietic stem cells (HSCs) may be transplanted into a subject suffering from cancer of the blood, or other blood or immune disorder. Examples of cancers of the blood that are potentially treated by hematopoietic cells or HSCs include: acute lymphoblastic leukemia, acute myeloblastic leukemia, chronic myelogenous leukemia (CML), Hodgkin's disease, multiple myeloma, and non-Hodgkin's lymphoma. Often, a subject suffering from such disease must undergo radiation and/or chemotherapeutic treatment in order to kill rapidly dividing blood cells. Introducing HSCs derived from the methods described herein to these subjects may help to repopulate depleted reservoirs of cells. In some cases, hematopoietic cells or HSCs derived by transdifferentiation may also be used to directly fight cancer. For example, transplantation of allogeneic HSCs has shown promise in the treatment of kidney cancer, see, e.g., Childs et al., (2000), N. Engl. J. Med., 343:750-758. In some embodiments, allogeneic, or even autologous, HSCs derived from induced cells may be introduced into a subject in order to treat kidney or other cancers. Hematopoietic cells or HSCs derived from induced cells may also be introduced into a subject in order to generate or repair cells or tissue other than blood cells, e.g., muscle, blood vessels, or bone. Such treatments may be useful for a multitude of disorders.

The number of administrations of treatment to a subject may vary. Introducing the purified batch of differentiated progeny cells into the subject may be a one-time event; but in certain situations, such treatment may elicit improvement for a limited period of time and require an on-going series of repeated treatments. In other situations, multiple administrations of the cells may be required before an effect is observed. The exact protocols depend upon the disease or condition, the stage of the disease and parameters of the individual subject being treated.

The cells may be introduced to the subject via any of the following routes: parenteral, intravenous, intraarterial, intramuscular, subcutaneous, transdermal, intratracheal, intraperitoneal, or into spinal fluid.

The purified batch of differentiated progeny cells may be administered in any physiologically acceptable medium. They may be provided alone or with a suitable substrate or matrix, e.g. to support their growth and/or organization in the tissue to which they are being transplanted. The cells may be introduced by injection, catheter, or the like. The cells may be frozen at liquid nitrogen temperatures and stored for long periods of time, being capable of use on thawing. If frozen, the cells will usually be stored in a 10% DMSO, 50% FCS, 40% RPMI 1640 medium. Once thawed, the cells may be expanded by use of growth factors and/or stromal cells associated with progenitor cell proliferation and differentiation.

While the in vitro experiments described above were performed using the frequencies, field intensities, and durations noted above, those parameters may be varied. For example, the frequency could be between 50 and 500 kHz, the electric field intensity could be between 0.5 and 5 V/cm; and the duration could be anything longer than 12 hours.

In the in vitro experiments using the Inovitro^{™} system described herein, the direction of the alternating electric fields was switched at one second intervals between two perpendicular directions. But in alternative embodiments, the direction of the alternating electric fields can be switched at a faster rate (e.g., at intervals between 1 and 1000 ms) or at a slower rate (e.g., at intervals between 1 and 100 seconds).

In the in vitro experiments using the Inovitro^{™} system described herein, the direction of the alternating electric fields was switched between two perpendicular directions by applying an AC voltage to two pairs of electrodes that are disposed 90° apart from each other in 2D space in an alternating sequence. But in alternative embodiments the direction of the alternating electric fields may be switched between two directions that are not perpendicular by repositioning the pairs of electrodes, or between three or more directions (assuming that additional pairs of electrodes are provided). For example, the direction of the alternating electric fields may be switched between three directions, each of which is determined by the placement of its own pair of electrodes. Optionally, these three pairs of electrodes may be positioned so that the resulting fields are disposed 90° apart from each other in 3D space. In other alternative embodiments, the direction of the field remains constant.

In the in vitro experiments using the Inovitro^{™} system described herein, the electrical field was capacitively coupled into the culture because the Inovitro^{™} system uses conductive electrodes disposed on the outer surface of the dish sidewalls, and the ceramic material of the sidewalls acts as a dielectric. But in alternative embodiments, the electric field could be applied directly to the cells without capacitive coupling (e.g., by modifying the Inovitro^{™} system configuration so that the conductive electrodes are disposed on the sidewall's inner surface instead of on the sidewall's outer surface).

The methods described herein can also be applied in the in vivo context by applying the alternating electric fields to a target region of a live subject's body to either prevent teratomas from developing or to treat teratomas that have already begun to develop. This may be accomplished, for example, using the Novocure Optune system (or a variant thereof) to apply alternating electric fields to a live subject's body. See, for example, US patent 7,565,205. In this situation, electrodes are positioned on the subject's body or implanted in the subject's body (e.g., just beneath the subject's skin, or in the vicinity of the target region), and AC voltages are applied between those electrodes to impose the alternating electric fields in the target region of the subject's body. Optionally, the orientation of the alternating electric fields may be periodically switched between two or more different directions.

In this in vivo situation, pluripotent stem cells are eliminated in vivo in order to prevent the formation of teratomas or to treat existing teratomas. This is accomplished by exposing the pluripotent stem cells to an alternating electric field for a period of time, the alternating electric field having a frequency and a field strength. The frequency and field strength of the alternating electric field are such that as a result of being exposed to the alternating electric field for the period of time, the pluripotent stem cells die off until the number of pluripotent stem cells is small enough to either (a) prevent the formation of a teratoma or (b) treat a teratoma that has already been formed. The frequency and field strength of the alternating electric field are such that differentiated cells in the subject's body that are exposed to the alternating electric field remain substantially unharmed.

### EXPERIMENTAL

The following examples are put forth so as to provide those of ordinary skill in the art with a complete disclosure and description of how to practice the methods described herein and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1

SEEDING OF CELLS ONTO COVERSLIPS. Human pluripotent stem cells (including human embryonic stem cells [ESCs] and human induced pluripotent stem cells [iPSCs]) were maintained in Essential 8 (E8) media supplemented with 10 µM ROCK Inhibitor Y-27632. ESCs (WA07 [H7] line) or iPSCs were seeded onto the center of a 22 mm diameter glass or plastic coverslip in 6-well plates. The coverslips had been coated with Matrigel diluted 1:200 in DMEM/F12 beforehand for at least one hour at 37° C in a conventional tissue culture incubator (37° C, 95% air, 5% CO₂). Once the cells adhered to the coverslip, a total of 2 mL of E8 media supplemented with 10 µM ROCK Inhibitor Y-27632 was added to each well. Media was changed on a daily basis for 1-2 days as the cells recovered prior to transfer of coverslips to the ceramic dishes of the Inovitro^{™} TTFields device (Novocure Inc., Haifa, Israel).

The procedure for seeding the human ESC- or iPSC-derived cardiomyocytes (ESC-CMs cr iPSC-CMs) was similar to that of the pluripotent stem cells except that the recovery period after seeding lasted longer (~5 days) until apparent spontaneous beating was observed. The ESC-CMs and iPSC-CMs were also maintained in RPMI 1640 medium containing B27 supplement plus insulin. A single monolayer of beating cardiac cells was visualized on the coverslips before starting the tumor-treating fields experiment. Imaging of cells was performed using a Leica DM IL LED inverted fluorescent microscope (Leica Microsystems, Buffalo Grove, IL) or a Revolve microscope (Echo laboratories, San Diego, CA).

TRYPAN BLUE CELL-COUNTING ASSAY USING AN AUTOMATED CELL COUNTER. Coverslips in ceramic dishes were prepared for cell counting by removing the media in addition to two washes of 2 mL of PBS. The coverslips were then transferred to a 6-well plate, whereupon 0.5 mL of Trypsin-LE (TrypLE) was added. Detached cells were suspended in a 15 mL Falcon Tube containing either 4.5 mL of E8 media supplemented with 10 µM ROCK Inhibitor Y-27632 (for ESCs and iPSCs) or RPMI 1640 media containing B27 supplement plus insulin (for ESC-CMs and iPSC-CMs). The cells were centrifuged at 300g for 5 minutes at room temperature. Once a pellet was obtained, the supernatant was aspirated and cells were resuspended in either 10 mL of Essential 8 or RPMI 1640 media for cell count.

After mixing the cell culture suspension, a 10 µL aliquot was taken and placed into a 1.5 mL Eppendorf tube. 10 µL of 0.4% Trypan Blue solution was then added to the cell culture aliquot, mixed by pipetting up and down, and loaded into one of the wells of the cell-counting slide. Cells were counted using a LUNA-FL Dual Fluorescence automated cell counter (Logos Biosystems, Annandale, VA, USA). Total live and dead cell counts were calculated and averaged from 3 technical replicates for each experimental condition (e.g., control [no TTF], 50 kHz, 100 kHz, 200 kHz, 300 kHz, 400 kHz and 500 kHz). The total cell counts were extrapolated based on a 1:10 dilution factor from original cell suspension.

QUANTITATIVE MEASURE OF CELL VIABILITY USING THE CELLTITER-GLO 2.0 ASSAY. Cellular viability of the human pluripotent stem cells and stem cell-derived cardiomyocytes was quantitatively measured using the CellTiter-Glo 2.0 assay (Promega, Madison, WI, USA). The CellTiter-Glo 2.0 assay provides a homogeneous method to determine the number of viable cells in culture by quantitating the amount of ATP present, which indicates the presence of metabolically active cells. Mono-oxygenation of luciferin is catalyzed by luciferase in the presence of Mg²⁺, ATP (which is contributed by viable cells), and molecular oxygen. A volume of CellTiter-Glo 2.0 reagent equal to the volume of cell culture medium present in each well was added (e.g., 0.5 mL of CellTiter-Glo 2.0 reagent was added to 0.5 mL of medium containing cells). The contents were mixed for 2 minutes on an orbital shaker to induce cell lysis. The 6-well plate was allowed to incubate at room temperature for 10 minutes to stabilize the luminescent signal. Afterwards, 50 µl from each well was transferred in triplicates to a white 96-well plate. The luminescence was recorded on a Synergy HTX multi-mode plate reader (BioTek, Winooski, VT, USA) using a 1 sec integration time. The BioTek Gen5 3.03 software was used to analyze the luminescence signal.

CONTRACTILITY ASSAY. Contractility measurements of the stem cell-derived cardiomyocytes were assessed by a Sony SI8000 Live Cell Imaging System (Sony Biotechnology, San Jose, CA) before and after applying the tumor treating fields. ESC-CMs or iPSC-CMs were treated at various alternating electric field frequencies (e.g., 50 kHz, 100 kHz, 200 kHz, 300 kHz, 400 kHz and 500 kHz) for 72 hours. The control dishes did not have any alternating electric field applied to them. Data was acquired using a high-performance video camera that utilized a unique motion vector software to capture the motion of cells with high temporal and spatial fidelity. Post-image acquisition, displacements and magnitudes of cellular motions were calculated using a motion detection algorithm developed by Sony Biotechnology. Regions of interest (ROIs) were positioned over single cells or clusters of cardiac cells and various contraction and relaxation parameters were calculated.

FLUORESCENCE-ACTIVATED CELL SORTING (FACS) AND CELL CYCLE ANALYSIS. For FACS and cell cycle analysis studies, we harvested the H7 ESCs after the specified alternating electric field experimental conditions, and washed them with PBS. We then fixed the cells in ice cold 70% ethanol for 2 h at -20° C. After fixation, we collected the cells by centrifugation and stained them with 1.0 mL of PBS containing 10 µg/mL propidium iodide (PI), 100 µg/mL RNase A, and 0.05% Triton X-100. The cells were incubated in PI solution for 15 min at room temperature in the dark and washed once with PBS, re-suspended in 0.5 ml PBS, and assessed for live and dead cells using the InCyte software in a Guava FACS analyzer (EMD Millipore, Burlington, MA), and for cell cycle status using the Guava cell cycle analysis software. We analyzed the results using FlowJo software (Tree Star, Ashland, OR) for measuring live/dead cells and cell cycle status.

### CULTURE AND MAINTENANCE OF HUMAN PLURIPOTENT STEM CELLS (hESCs and hiPSCs)

GENERATION OF iPSCs. Dermal fibroblasts from a healthy human donor were reprogrammed into iPSCs using non-integrating Sendai virus vectors carrying the following transcription factors: Oct4, Sox2, Nanog, and cMyc.

ESC CULTURE AND MAINTENANCE. The human ESC line used in these experiments was the WA07 (H7) line transfected with a lentivirus vector that expresses Luciferase and Tomato Red under the EFla promoter. The ESCs were grown to 90% confluence on Matrigel coated plates (ES Qualified, BD Biosciences, San Diego, CA) using a chemically defined E8 medium as previously described. The medium was changed on a daily basis, and cells were passaged every 3-4 days with EDTA (Thermo Fisher Scientific, CA). 10 µM ROCK Inhibitor Y-27632 was supplemented to dissociated cells prior to plating to prevent apoptosis.

CARDIAC DIFFERENTIATION. Human pluripotent stem cells were grown to 90% confluence and subsequently differentiated into beating cardiomyocytes as previously described in Burridge et al. (2014); Burridge et al. (2015). Cardiac cell purity of 80-90% was obtained based on flow cytometry in which cells were stained for cardiac markers such as troponin T and α actinin. In brief, on day 0, cells were supplemented with a basal medium (RPMI 1640 [Thermo Fisher Scientific] and 2% B27 supplement minus insulin [Thermo Fisher Scientific]), with the addition of 6 µM of CHIR-99021 [Selleck Chemicals], a selective inhibitor of glycogen synthase kinase 3β, which activates the canonical Wnt signaling pathway. On day 2, the medium was replaced with basal medium without CHIR99021 supplementation. On day 3, cells were treated with 5 µM of IWR-1 [Selleck Chemicals], a Wnt antagonist, for 2 days. On day 5 and every subsequent other day until harvest, the medium was replaced with fresh basal medium. 10X TrypLE was added to the cardiomyocytes for 10 minutes at 37° C to dissociate into single cells. Following dissociation, ESC-CMs or iPSC-CMs were re-plated on freshly Matrigel coated 6-well plates in RPMI 1640 medium supplemented with 10% Knock out serum supplement, B27 supplement with insulin, and 1µM of Thiazovivin.

CELL INJECTIONS IN MICE. Female immunodeficient nude (NU/NU) mice were anesthetized with 2% isoflurane and subcutaneously injected with approximately 500,000 H7 ESCs. The H7 ESCs were suspended in 100 µL Matrigel before being injected into the right flanks of the mice using a 28-gauge syringe needle. Moreover, the alternating electric field-treated H7 ESCs (i.e., those treated at 300 kHz for 3-4 days) were subcutaneously injected in the left flanks of the mice. A Matrigel only injection in the left shoulder blade was used as our control. Syringes were kept on ice prior to injection to prevent solidification of Matrigel at room temperature. The Stanford Administrative Panel on Laboratory Animal Care (APLAC) approved all animal procedures.

BIOLUMINESCENCE IMAGING (BLI) OF TRANSPLANTED CELLS TO ASSESS CELL SURVIVAL AND TERATOMA FORMATION. BLI was performed to track cell proliferation during the course of this study. In-vivo BLI was performed on the IVIS Spectrum imaging system (Xenogen Corporation, Alameda, CA) for up to 5-weeks post-cell injection. Cell survival and proliferation were monitored at day 0, 1, 3, 7, and every 7 days for up to 35 days post-cell transplantation. 1 gram of D-Luciferin Firefly potassium salt was diluted in 23 mL PBS, and 300 µl of this mixture was administered intraperitoneally using a 28-gauge insulin syringe needle. Ten minutes after intraperitoneal injection, animals were imaged for 20 minutes using 1 second to 5 min acquisition windows. Living Image software (Caliper LifeSciences, version 4.3.1) was used to analyze the bioluminescent images at different time points. ROIs were drawn over the sites of cell injection and the control region. The luminescence signal was quantified in radiance units of photons per second per square centimeter per steradian (photons/sec/cm²/sr).

TERATOMA EXPLANTATION AND HISTOLOGY. After the teratoma grew to a size of about 15 mm in diameter, the mice were euthanized and the teratomas were excised, fixed with 4% paraformaldyhyde, and sent to a pathological core lab for paraffin sectioning and H&E staining.

STATISTICAL ANALYSIS. Statistical analysis was performed using GraphPad Prism (version 7.04) and SPSS (IBM, version 21). Tests that had an alpha level for significance set at P < 0.05 were considered significant. Data are reported as mean ± standard deviation unless otherwise noted.

LIST OF MATERIALS. The following materials were used in the experiments described herein: Falcon 6-Well Clear Flat Bottom Plate (Corning, cat. no.: 353046); Essential 8^{™} Medium (ThermoFisher Scientific, cat. no.: A1517001); RPMI 1640 Medium (ThermoFisher Scientific, cat. no.: 11875093); Phosphate Buffered Saline, 10X Solution, Fisher BioReagents^{™} (Thermo Fisher Scientific, cat. no.: BP3994); CHIR-99021 (Selleck, cat. no.: S2924); IWR-1 (Selleck, cat. no.: S7086); B-27^{™} Supplement (50X), serum free (ThermoFisher Scientific, cat. no.: 17504044); B-27^{™} Supplement, minus insulin (ThermoFisher Scientific, cat. no.: A1895601); Corning Matrigel Growth Factor Reduced (ThermoFisher Scientific, cat no.: 356231); TrypLE^{™} Select Enzyme (10X), no phenol red (ThermoFisher Scientific, cat. no.: A1217701); BD Lo-Dose U-100 Insulin Syringes 0.5mL (ThermoFisher Scientific, cat. no.: 329461); CellTiter-Glo 2.0 (Promega, cat. no.: G9242); Paraformaldeyde 20% Solution EM Grade (Electron Microscopy Sciences, cat. no.: 15713-S); Propidium iodide (Sigma-Aldrich, cat. no.: P4170 ); RNase A (Thermo Fisher Scientific, cat. no.: EN0531); Triton X-100 (Sigma-Aldrich, cat. no.: X100); XenoLight D-Luciferin Potassium Salt (PerkinElmer, cat. no.: 122799); DM IL LED inverted fluorescent microscope (Leica Microsystems, Buffalo Grove, IL); Revolve Microscope (Echo laboratories, San Diego, CA); Sony SI8000 Live Cell Imaging System (Sony Biotechnology, San Jose, CA).; Synergy HTX multi-mode plate reader (BioTek, Winooski, VT, USA); LUNA-FL Dual Fluorescence automated cell counter (Logos Biosystems, Annandale, VA, USA).; Guava easyCyte HT Flow Cytometer (EMD Millipore, Burlington, MA); Inovitro^{™} in-vitro TTField device (Novocure Inc., Haifa, Israel).

### REFERENCES

Takahashi K, Yamanaka S. Induction of pluripotent stem cells from mouse embryonic and adult fibroblast cultures by defined factors. Cell. 2006;126:663-676.
Takahashi K, Tanabe K, Ohnuki M, et al. Induction of pluripotent stem cells from adult human fibroblasts by defined factors. Cell. 2007;131:861-872.
Liang P, Sallam K, Wu H, et al. Patient-Specific and Genome-Edited Induced Pluripotent Stem Cell-Derived Cardiomyocytes Elucidate Single-Cell Phenotype of Brugada Syndrome. J Am Coll Cardiol. 2016;68:2086-2096.
Matsa E, Burridge PW, Yu KH, et al. Transcriptome Profiling of Patient-Specific Human iPSC-Cardiomyocytes Predicts Individual Drug Safety and Efficacy Responses In Vitro. Cell Stem Cell. 2016;19:311-325.
Burridge PW, Li YF, Matsa E, et al. Human induced pluripotent stem cell-derived cardiomyocytes recapitulate the predilection of breast cancer patients to doxorubicin-induced cardiotoxicity. Nat Med. 2016;22:547-556.
Churko JM, Lee J, Ameen M, et al. Transcriptomic and epigenomic differences in human induced pluripotent stem cells generated from six reprogramming methods. Nature Biomedical Engineering. 2017;1:826-837.
Lan F, Lee AS, Liang P, et al. Abnormal calcium handling properties underlie familial hypertrophic cardiomyopathy pathology in patient-specific induced pluripotent stem cells. Cell Stem Cell. 2013;12:101-113.
Sun N, Panetta NJ, Gupta DM, et al. Feeder-free derivation of induced pluripotent stem cells from adult human adipose stem cells. Proc Natl Acad Sci U S A. 2009;106:15720-15725.
Chen G, Gulbranson DR, Hou Z, et al. Chemically defined conditions for human iPSC derivation and culture. Nat Methods. 2011;8:424-429.
Burridge PW, Matsa E, Shukla P, et al. Chemically defined generation of human cardiomyocytes. Nat Methods. 2014;11:855-860.
Burridge PW, Holmstrom A, Wu JC. Chemically Defined Culture and Cardiomyocyte Differentiation of Human Pluripotent Stem Cells. Curr Protoc Hum Genet. 2015;87:2123-2115.
Ebert AD, Kodo K, Liang P, et al. Characterization of the molecular mechanisms underlying increased ischemic damage in the aldehyde dehydrogenase 2 genetic polymorphism using a human induced pluripotent stem cell model system. Sci Transl Med. 2014;6:255ra130.
Lee AS, et al. Effects of cell number on teratoma formation by human embryonic stem cells. Cell Cycle. 2009;8:2608-2612.
Tan HL, Fong WJ, Lee EH, Yap M, Choo A. mAb 84, a cytotoxic antibody that kills undifferentiated human embryonic stem cells via oncosis. Stem Cells. 2009;27:1792-1801.
Choo AB, et al. Selection against undifferentiated human embryonic stem cells by a cytotoxic antibody recognizing podocalyxin-like protein-1. Stem Cells. 2008;26:1454-1463.
Tang C, et al. An antibody against ssea-5 glycan on human pluripotent stem cells enables removal of teratoma-forming cells. Nature Biotechnology. 2011;29:829-834.
Fong CY, et al. Separation of SSEA-4 and TRA-1-60 labelled undifferentiated human embryonic stem cells from a heterogeneous cell population using magnetic-activated cell sorting (MACS) and fluorescence-activated cell sorting (FACS). Stem Cell Reviews and Reports. 2009;5:72-80.
Blum B, Bar-Nur O, Golan-Lev T, Benvenisty N. The anti-apoptotic gene survivin contributes to teratoma formation by human embryonic stem cells. Nature Biotechnology. 2009;27:281-287.
Schuldiner M, Itskovitz-Eldor J, Benvenisty N. Selective ablation of human embryonic stem cells expressing a "suicide" gene. Stem cells. 2003;21:257-265.
Lee MO, et al. Inhibition of pluripotent stem cell-derived teratoma formation by small molecules. Proceedings of the National Academy of Sciences of the United States of America. 2013;110:E3281-3290.
Ben-David U, et al. Selective elimination of human pluripotent stem cells by an oleate synthesis inhibitor discovered in a high-throughput screen. Cell Stem Cell. 2013;12:167-179
Lin YT, et al. Elimination of undifferentiated human embryonic stem cells by cardiac glycosides. Scientific Reports. 2017;7:5289
Lee AS, et al. Brief report: External beam radiation therapy for the treatment of human pluripotent stem cell-derived teratomas. Stem Cells. 2017;35:1994-2000.
Lee AS, Tang C, Rao MS, Weissman IL, Wu JC. Tumorigenicity as a clinical hurdle for pluripotent stem cell therapies. Nature Medicine. 2013;19:998-1004.

While the present invention has been disclosed with reference to certain embodiments, numerous modifications, alterations, and changes to the described embodiments are possible without departing from the scope of the present invention, as defined in the appended claims. Accordingly, it is intended that the present invention not be limited to the described embodiments, but that it has the full scope defined by the language of the following claims.

## Claims

1. A method of eliminating residual pluripotent stem cells from a batch of
differentiated progeny cells in order to prevent the formation of teratomas in a stem cell-based therapy, the method **characterized by**:
exposing, in vitro, the batch of differentiated progeny cells and the residual pluripotent stem cells to an alternating electric field for a period of time, the alternating electric field having a frequency and a field strength,
wherein the frequency and the field strength of the alternating electric field are such that, as a result of being exposed to the alternating electric field for the period of time, the pluripotent stem cells die off, resulting in a purified batch of differentiated progeny cells that is rendered safe for subsequent use in the stem cell-based therapy, and
wherein the frequency and the field strength of the alternating electric field are such that the differentiated cells that are exposed to the alternating electric field for the period of time remain substantially unharmed.

2. The method of claim 1, wherein the purified batch contains fewer than 100,000 pluripotent stem cells.

3. The method of claim 1, wherein the purified batch contains fewer than 10,000 pluripotent stem cells.

4. The method of claim 1, wherein the period of time is at least 12 hours.

5. The method of claim 1, wherein the period of time is at least 2 days.

6. The method of claim 1, wherein the alternating electric field has an orientation that is switched from time to time between at least two different directions during the period of time.

7. The method of claim 1, wherein the frequency is between 50 kHz and 500 kHz.

8. The method of claim 7, wherein the field strength is at least 1 V/cm.

9. The method of claim 1, wherein the frequency is between 250 kHz and 350 kHz and the field strength is at least 1 V/cm.

10. The method of claim 1, wherein the frequency is between 50 kHz and 500 kHz, the field strength is at least 1 V/cm, and the period of time is at least 12 hours.

11. The method of claim 1, wherein the frequency is between 250 kHz and 350 kHz, the field strength is at least 1 V/cm, and the period of time is at least 3 days.

12. The method of claim 1, wherein, prior to the exposing, the batch of differentiated progeny cells is obtained by expanding pluripotent stem cells and differentiating the expanded pluripotent stem cells into the batch of differentiated progeny cells.

13. The method of claim 1, wherein the pluripotent stem cells comprise one or more of induced pluripotent stem cells, embryonic stem cells, cancer stem cells, and embryonic germ cells.

14. The method of claim 1, wherein the differentiated progeny cells are comprised of cardiomyocytes or cardiomyocyte progenitors.

15. The method of claim 1, wherein the pluripotent stem cells and the differentiated progeny cells are human cells.

## Patentansprüche

1. Verfahren zur Beseitigung restlicher pluripotenter Stammzellen aus einer Charge differenzierter Nachkommenzellen, um die Bildung von Teratomen in einer stammzellenbasierten Therapie zu verhindern, wobei das Verfahren **gekennzeichnet ist durch**:
Aussetzen der Charge differenzierter Nachkommenzellen und der restlichen pluripotenten Stammzellen in vitro für einen Zeitraum gegenüber einem elektrischen Wechselfeld, wobei das elektrische Wechselfeld eine Frequenz und eine Feldstärke aufweist,
wobei die Frequenz und die Feldstärke des elektrischen Wechselfeldes derart sind, dass infolge des Ausgesetztseins gegenüber dem elektrischen Wechselfeld für den Zeitraum die pluripotenten Stammzellen absterben, was zu einer gereinigten Charge differenzierter Nachkommenzellen führt, die zur anschließenden Verwendung in der stammzellenbasierten Therapie sicher gemacht wird, und
wobei die Frequenz und die Feldstärke des elektrischen Wechselfeldes derart sind, dass die differenzierten Zellen, die dem elektrischen Wechselfeld für den Zeitraum ausgesetzt werden, im Wesentlichen unbeschädigt bleiben.

2. Verfahren nach Anspruch 1, wobei die gereinigte Charge weniger als 100 000 pluripotente Stammzellen enthält.

3. Verfahren nach Anspruch 1, wobei die gereinigte Charge weniger als 10 000 pluripotente Stammzellen enthält.

4. Verfahren nach Anspruch 1, wobei der Zeitraum mindestens 12 Stunden beträgt.

5. Verfahren nach Anspruch 1, wobei der Zeitraum mindestens 2 Tage beträgt.

6. Verfahren nach Anspruch 1, wobei das elektrische Wechselfeld eine Orientierung aufweist, die während des Zeitraums von Zeit zu Zeit zwischen mindestens zwei verschiedenen Richtungen gewechselt wird.

7. Verfahren nach Anspruch 1, wobei die Frequenz zwischen 50 kHz und 500 kHz liegt.

8. Verfahren nach Anspruch 7, wobei die Feldstärke mindestens 1 V/cm beträgt.

9. Verfahren nach Anspruch 1, wobei die Frequenz zwischen 250 kHz und 350 kHz liegt und die Feldstärke mindestens 1 V/cm beträgt.

10. Verfahren nach Anspruch 1, wobei die Frequenz zwischen 50 kHz und 500 kHz liegt, die Feldstärke mindestens 1 V/cm beträgt und der Zeitraum mindestens 12 Stunden beträgt.

11. Verfahren nach Anspruch 1, wobei die Frequenz zwischen 250 kHz und 350 kHz liegt, die Feldstärke mindestens 1 V/cm beträgt und der Zeitraum mindestens 3 Tage beträgt.

12. Verfahren nach Anspruch 1, wobei die Charge differenzierter Nachkommenzellen vor dem Aussetzen durch Expandieren pluripotenter Stammzellen und Differenzieren der expandierten pluripotenten Stammzellen in die Charge differenzierter Nachkommenzellen erhalten wird.

13. Verfahren nach Anspruch 1, wobei die pluripotenten Stammzellen eines oder mehrere von induzierten pluripotenten Stammzellen, embryonalen Stammzellen, Krebsstammzellen und embryonalen Keimzellen umfassen.

14. Verfahren nach Anspruch 1, wobei die differenzierten Nachkommenzellen Kardiomyozyten oder Kardiomyozyten-Vorläufer umfassen.

15. Verfahren nach Anspruch 1, wobei die pluripotenten Stammzellen und die differenzierten Nachkommenzellen menschliche Zellen sind.

## Revendications

1. Procédé d'élimination des cellules souches pluripotentes résiduelles d'un lot de cellules progénitrices différenciées afin de prévenir la formation de tératomes dans le cadre d'une thérapie à base de cellules souches, le procédé étant **caractérisé par** les étapes suivantes :
exposer, in vitro, le lot de cellules progénitrices différenciées et les cellules souches pluripotentes résiduelles à un champ électrique alternatif pendant une certaine période de temps, le champ électrique alternatif ayant une fréquence et une intensité de champ,
dans lequel la fréquence et l'intensité du champ électrique alternatif sont telles que, en conséquence de l'exposition au champ électrique alternatif pendant la période de temps, les cellules souches pluripotentes meurent, ce qui résulte en un lot purifié de cellules progénitrices différenciées qui est rendu sûr pour une utilisation ultérieure dans la thérapie à base de cellules souches, et
dans lequel la fréquence et l'intensité du champ électrique alternatif sont telles que les cellules différenciées qui sont exposées au champ électrique alternatif pendant la période de temps restent sensiblement indemnes.

2. Procédé selon la revendication 1, dans lequel le lot purifié contient moins de 100 000 cellules souches pluripotentes.

3. Procédé selon la revendication 1, dans lequel le lot purifié contient moins de 10 000 cellules souches pluripotentes.

4. Procédé selon la revendication 1, dans lequel la période de temps est d'au moins 12 heures.

5. Procédé selon la revendication 1, dans lequel la période de temps est d'au moins 2 jours.

6. Procédé selon la revendication 1, dans lequel le champ électrique alternatif a une orientation qui est commutée de temps en temps entre au moins deux directions différentes pendant la période de temps.

7. Procédé selon la revendication 1, dans lequel la fréquence est comprise entre 50 kHz et 500 kHz.

8. Procédé selon la revendication 7, dans lequel l'intensité du champ est d'au moins 1 V/cm.

9. Procédé selon la revendication 1, dans lequel la fréquence est comprise entre 250 kHz et 350 kHz et l'intensité du champ est d'au moins 1 V/cm.

10. Procédé selon la revendication 1, dans lequel la fréquence est comprise entre 50 kHz et 500 kHz, l'intensité du champ est d'au moins 1 V/cm, et la période de temps est d'au moins 12 heures.

11. Procédé selon la revendication 1, dans lequel la fréquence est comprise entre 250 kHz et 350 kHz, l'intensité du champ est d'au moins 1 V/cm, et la période de temps est d'au moins 3 jours.

12. Procédé selon la revendication 1, dans lequel, avant l'exposition, le lot de cellules progénitrices différenciées est obtenu par expansion de cellules souches pluripotentes et différenciation des cellules souches pluripotentes expansées en un lot de cellules progénitrices différenciées.

13. Procédé selon la revendication 1, dans lequel les cellules souches pluripotentes comprennent une ou plusieurs des cellules souches pluripotentes induites, des cellules souches embryonnaires, des cellules souches cancéreuses et des cellules germinales embryonnaires.

14. Procédé selon la revendication 1, dans lequel les cellules progénitrices différenciées sont constituées de cardiomyocytes ou de progéniteurs de cardiomyocytes.

15. Procédé selon la revendication 1, dans lequel les cellules souches pluripotentes et les cellules progénitrices différenciées sont des cellules humaines.
